(19) Europäisches Patentamt
European Patent Office
Office européen des brevets

(11) **EP 2 353 644 B1**

(12) **EUROPÄISCHE PATENTSCHRIFT**

(45) Veröffentlichungstag und Bekanntmachung des Hinweises auf die Patenterteilung:
**04.07.2012 Patentblatt 2012/27**

(51) Int Cl.:
***A61N 1/39*** *(2006.01)*   ***A61B 5/0464*** *(2006.01)*

(21) Anmeldenummer: **11151199.4**

(22) Anmeldetag: **18.01.2011**

(54) **Kardioverter/Defibrillator und Signalverarbeitungsvorrichtung zur Klassifikation intrakardialer Signale**

Cardioverter/defibrillator and signal processing device for classifying intracardiac signals

Cardioverteur/défibrillateur et dispositif de traitement de signal pour la classification de signaux intra-cardiaux

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.02.2010 US 302569 P**

(43) Veröffentlichungstag der Anmeldung:
**10.08.2011 Patentblatt 2011/32**

(73) Patentinhaber: **BIOTRONIK SE & Co. KG**
**12359 Berlin (DE)**

(72) Erfinder:
• **Stäuber, Philipp**
**10961, Berlin (DE)**

• **Neumann, Andreas**
**12437, Berlin (DE)**
• **Busch, Ulrich**
**10318, Berlin (DE)**
• **Wohlgemuth, Peter**
**09123, Chemnitz (DE)**

(74) Vertreter: **Lindner-Vogt, Karin L.**
**Biotronik SE & Co. KG**
**Corporate Intellectual Properties**
**Woermannkehre 1**
**12359 Berlin (DE)**

(56) Entgegenhaltungen:
**EP-A2- 0 848 965      US-A1- 2003 032 988**
**US-A1- 2005 137 485**

**Beschreibung**

Die Erfindung betrifft eine Signalverarbeitungsvorrichtung zur Klassifikation intrakardialer Signale und einen Herzstimulator, insbesondere einen Kardioverter/Defibrillator mit einer solchen Signalverarbeitungsvorrichtung.

Der Herzstimulator kann darüber hinaus auch als Einkammerherzstimulator, als biventrikulärer Herzstimulator oder dergleichen ausgebildet sein.

Implantierbare Herzstimulatoren in Form von Herzschrittmachern oder Kardiovertern/Defibrillatoren sind grundsätzlich bekannt. Solche Herzstimulatoren sind in der Regel an Elektrodenleitungen angeschlossen, die in einer Kammer eines Herzens oder in unmittelbarer Nähe Stimulations- oder Defibrillationselektroden besitzen.

Derartige Herzstimulatoren dienen der Behandlung von Störungen der natürlichen Kontraktion der Kammern eines Herzens mit Hilfe von Stimulationsimpulsen und/oder Defibrillationsschocks.

In der Kammer eines Herzens breitet sich eine lokale Erregung des Herzmuskelgewebes (Myokards) von einem Erregungsort ausgehend per Reizleitung im Myokard aus und führt zu einer Depolarisation der Muskelzellen und damit zu einer Kontraktion des Myokards. Nach kurzer Zeit kommt es zu einer Repolarisation der Muskelzellen und damit zu einer Entspannung des Myokards. Während der Phase der Depolarisation sind die Herzmuskelzellen für eine Erregung unempfindlich, d.h. refraktär. Die mit der Depolarisation und Repolarisation einhergehenden elektrischen Potentiale können wahrgenommen und deren zeitlicher Verlauf - als Elektrokardiogramm bezeichnet - ausgewertet werden.

Das Erfassen solcher natürlichen (intrisischen) Ereignisse erfolgt durch Ableiten der elektrischen Potenziale des Myokards der jeweiligen Herzkammer mit Hilfe von Sensing-Elektroden, die Teil einer entsprechenden Elektrodenleitung sind. Dabei können die Sensing-Elektroden gleichzeitig die Stimulationselektroden sein und abwechselnd als Stimulations- und als Sensing-Elektroden verwendet werden. Typischer Weise ist für das Sensing - d.h. die Wahrnehmung intrinsischer Ereignisse - ein Sensing-Elektrodenpaar vorgesehen, das von zwei benachbarten Elektroden, nämlich einer Spitzenelektrode (Tip-Elektrode) und einer Ringelektrode gebildet ist, von denen die Spitzenelektrode auch als Stimulationselektrode dient. Auf diese Weise erfolgt eine bipolare Ableitung eines intrakardialen Elektrokardiogramms (IEGM).

Üblicherweise erfassen die Elektroden elektrische Signale der Herzkammer, in der sie angeordnet sind. Zusätzlich ist auch die Erfassung von so genannten Fernfeld Elektrokardiogrammen möglich. Als Fernfeld Elektrokardiogramm werden elektrische Potentiale des Myokards bezeichnet, deren Ursprung von der Position der Elektrode entfernt liegt. Beispielsweise können mit einer im Ventrikel platzierten Elektrode auch Signale eines Atriums erfasst werden. Fernfeld Elektrokardiogramme können nicht nur mit den für die Stimulation vorgesehenen Elektroden, sondern auch mit den Schockwendeln, die für die Abgabe von Defibrillationsschocks vorgesehen sind, erfasst werden. Der Gegenpol für die Signalerfassung kann eine andere Elektrode, eine andere Schockwendel oder auch das elektrisch leitende Gehäuse eines implantierbaren Herzstimulators sein.

Die Sensing-Elektroden sind im Betrieb des Herzstimulators mit entsprechenden Sensing-Einheiten verbunden, die ausgebildet sind, ein jeweiliges über eine Sensing-Elektrode (bzw. ein Sensing-Elektrodenpaar) aufgenommenes Elektrokardiogramm auszuwerten und insbesondere intrinsische atriale bzw. ventrikuläre Ereignisse zu detektieren, d.h. natürliche atriale oder ventrikuläre Kontraktionen. Dies geschieht beispielsweise durch Schwellwertvergleich, d.h. ein intrinsisches Ereignis wird detektiert, wenn ein jeweiliges intrakardiales Elektrokardiogramm einen geeignet vorgegebenen Schwellwert überschreitet.

Aus der zeitlichen Abfolge atrialer bzw. ventrikulärer Ereignisse, kann die jeweilige intrinsische atriale Herzrate (atriale Frequenz) bzw. ventrikuläre Herzrate (ventrikuläre Frequenz) bestimmt werden.

Ein implantierbarer Kardioverter/Defibrillator besitzt über die bereits beschriebenen Eigenschaften eines Herzschrittmachers hinaus weiterhin die Möglichkeit, einen stärkeren Stromimpuls an das Herz abzugeben, der nicht nur einen kleinen Teil des Myokards stimulieren (depolarisieren) soll, sondern einen möglichst großen Teil des Myokards depolarisieren und damit refraktär machen soll, um so eine für Fibrillationen typische kreisende Erregung des Myokards zu unterbrechen. Ein derartiger Impuls wird als Defibrillationsschock bezeichnet. Er wird typischer Weise über eine im Vergleich zur Stimulations- oder Sensing-Elektrode großflächige Defibrillationselektrode abgegeben.

Solch eine Defibrillationselektrode ist häufig in Form einer Schockwendel auf der Außenfläche der Elektrodenleitung in der jeweiligen Herzkammer realisiert. Beispielsweise kann eine ventrikuläre Elektrodenleitung neben einer Tip-Elektrode oder einer Ringelektrode für die Stimulation und das Sensing auch eine ventrikuläre Schockwendel und darüber hinaus auch eine nach Implantation in der Vena Cava Superior befindliche proximale Schockwendel besitzen.

Die Abgabe eines Defibrillationsschocks erfolgt in der Regel dann, wenn der Herzstimulator eine Fibrillation, d.h. eine unregelmäßige hochfrequente Eigenaktivität des Herzens, detektiert, die auch als Kammerflimmern bezeichnet wird, und die dazu führt, dass es zu keiner vollständigen Kontraktion der betroffenen Herzkammer kommt. Eine solche Fibrillation zählt zu den tachykarden Arrhythmien, zu denen neben Fibrillationen auch Tachykardien (z.B. Kammerflattern) zählen. Im Unterschied zu einer Fibrillation folgt bei einer Tachykardie regelmäßig eine vollständige Kontraktion der betroffenen Herzkammer, jedoch mit einer Rate, die höher ist als physiologisch angemessen. Häufig können solche Tachykardien durch eine antitachykarde Stimulation behandelt werden und bedürfen nicht eines Defibrillationsschocks.

Fibrillationen werden in der Regel mit einem Defibrillationsschock behandelt.

Zur Detektion von Kammerflimmern (ventrikulärer Fibrillation) ist typischerweise eine Detektionseinrichtung als Bestandteil einer Steuereinheit des Herzstimulators vorgesehen, die mit der rechtsventrikulären Sensing-Einheit verbunden und ausgebildet ist, eine primäre Kammerflimmerdetektion auf Basis des rechtsventrikulären, bipolar abgeleiteten intrakardialen Elektrokardiogramms (IEGM) durchzuführen. Ist eine vorgegebene Detektionsbedingung (X aus Y-Kriterium) erfüllt, zeigt die Detektionseinrichtung eine ventrikuläre Fibrillation an.

Bei Tachykardien des Ventrikels unterscheidet man zwischen übergeleiteten supraventrikulären Tachykardien (SVT) und ventrikulären Tachykardien (VT). Letztere haben ihren Ursprung im Ventrikel selbst, während supraventrikuläre Tachykardien ihren Ursprung im Atrium haben. Für die nach Detektion einer Tachykardie eingeleitete Therapie ist die Art der ventrikulären Tachykardie (ventrikuläre Tachykardie (VT) oder supraventrikuläre Tachykardie (SVT)) von Bedeutung.

Die Behandlung von Tachykardien und Fibrillationen mittels intrakardialer Elektrotherapie kann auf verschiedene bekannte Arten und Weisen erfolgen. Bekannt ist eine antitachykarde Stimulation (anti tachycardia pacing, ATP) in Form einer Overdrive-Stimulation, bei der Stimulationsimpulse mit einer gegenüber der vorliegenden, intrinsischen (tachykarden) Herzrate erhöhten Stimulationsrate abgegeben werden, die Abgabe von Kardioversionsschocks oder die Abgabe von Defibrillationsschocks, wobei erstere üblicherweise eine geringere Energie haben als letztere. Defibrillationsschocks sollen das gesamte Myokard einer betroffenen Herzkammer gleichzeitig refraktär und damit für eine Erregung temporär unempfänglich machen, um so einen kreisende Erregung des betroffenen Herzmuskels zu unterbrechen.

Die Behandlung ventrikulärer Tachykardien (VT) durch Overdrive-Stimulation im Rahmen eines antitachykarden pacings (ATP) soll bewirken, dass durch einen Stimulationsimpuls, der vor der natürlichen (intrinsischen) Erregung der betroffenen Herzkammer erfolgt, ein für ventrikuläre Tachykardien (VT, Kammerflattern) typischer Wiedereintritts-Zyklus (Reentry-Zyklus) der Erregung des Myokards unterbrochen wird. Hierzu ist einer zuverlässige Erfassung intrinsischer Ventrikelkontraktionen vor dem Auslösen der ATP erforderlich.

Die Klassifikation mit den bisherigen Methoden zur SVT/VT-Diskriminierung bei Einkammer-ICDs führt häufig zu inadäquaten Therapien, weil die Auswertung der IEGMs nur hinsichtlich der Intervalle bzw. deren Änderungen durchgeführt wird.

Da eine maximale Sensitivität immer Priorität hat (echte VTs sollen immer erkannt werden), ist, die Spezifität (= Erkennung von SVTs) bei den bisherigen Verfahren oft nicht zufriedenstellend. SVTs, die fälschlicherweise als VTs klassifiziert werden, führen zu einer nicht unerheblichen Anzahl inadäquater Defibrillator-Schocks, die die betreffenden Patienten psychisch und physisch belasten. Auch bei inadäquaten ATP-Therapien besteht die Gefahr, dadurch eine gefährliche ventrikuläre Tachykardie auszulösen.

Der nächstliegende Stand der Technik wird im Document EP-A-0848965 offenbart.

Mit morphologischen Verfahren kann die Spezifität der SVT/VT-Diskriminierung verbessert werden, ohne die Sensitivität signifikant einzuschränken.

Aufgabe der Erfindung ist es, eine Vorrichtung zu schaffen, die die Erkennungsleistung (SVT/VT-Diskriminierung) bei der Unterscheidung ventrikulärer Tachykardien (VT), bei denen Therapie durch einen Kardioverter/Defibrillator notwendig ist, von supraventrikulären Tachykardien (SVT), z.B. übergeleitetes atriales Flattern/Flimmern oder übergeleitete Sinustachykardie, bei denen keine ICD-Therapie notwendig ist, gegenüber vorbekannten Lösungen verbessert. Die Vorrichtung soll in ICDs einsetzbar sein, d.h. Rechenaufwand und Stromverbrauch müssen angemessen sein. Bevorzugtes Ziel ist eine automatische Klassifikation, welche lernfähig ist, patienten-individuell arbeitet und möglichst keine vom Arzt einzustellenden parameter beinhaltet.

Die Klassifikation soll bevorzugt für Einkammer- und/oder Mehrkammer-Kardioverter/Defibrillatoren geeignet sein.

Erfindungsgemäß wird die erstgenannte Aufgabe durch eine Signalverarbeitungsvorrichtung zur Klassifikation intrakardialer Signale gelöst, die einen Signaleingang für ein physiologisches Signal, insbesondere ein Elektrokardiogramm, eine Morphologieerfassungseinheit, die mit dem Signaleingang zumindest indirekt verbunden ist, und eine Morphologie-Diskriminierungseinheit aufweist.

Die Morphologieerfassungseinheit ist ausgebildet

- Signalmaxima und -minima in einem Signalabschnitt als Spitzenamplituden dieses Signalabschnitts zu detektieren und zu einer vorgegebenen Zahl von Signalmaxima und -minima jeweils einen zur Größe der Spitzenamplitude proportionalen Spitzenwert zu bestimmen, und

- aus zwei benachbarten Spitzenwerten mit entgegengesetzter Polarität einen Spitzen-Spitzen-Wert erster Art als Summe der Absolutwerte der einzelnen Spitzenwerte zu bestimmen und

- im selben Signalabschnitt weitere Spitzen-Spitzen-Werte erster Art als Summe der Absolutwerte benachbarter Spitzenwerte mit entgegengesetzter Polarität zu bestimmen,

- und die Spitzen-Spitzen-Werte auf den größten der bestimmten Spitzen-Spitzen-Wert zu normieren und so eine vorgegebene Anzahl normierter Spitzen-Spitzen-Werte zu bilden, welche zusammen einen Morphologie-Vektor ergeben, der für die Morphologie eines jeweiligen Signalabschnitts charakteristisch ist.

Signalmaximum oder -minimum ist jeweils derjenige Signalwert zwischen zwei Nulldurchgängen des Signals, der den größten Absolutwert besitzt. Ein Signalminimum hat ein negatives Vorzeichen und ein Signalmaximum ein positives Vorzeichen. Der Spitzen-Spitzen-Wert ergibt sich durch Addition des Absolutwertes des Amplitudenwertes des Signalmaximums und des Absolutwertes des Amplitudenwertes des Signalminimums. Das Signalmaximum und das Signalminimum befinden sich dabei typischerweise in zwei unmittelbar benachbarten Teilabschnitten des Signalabschnitts, die durch einen Nulldurchgang des Signals voneinander getrennt sind.

Bevorzugte weitere Elemente des Morphologievektors sind beispielsweise Spitzen-Spitzen-Werte zweiter Art als Differenz der Absolutwerte benachbarter Spitzenwerte gleicher Polarität, insbesondere dann, wenn ein Teilabschnitt des Signalabschnitts zu finden, benachbarte lokale Signalmaxima und/oder benachbarte lokale Signalminima enthält.

Die Morphologie-Diskriminierungseinheit ist mit der Morphologieerfassungseinheit verbunden und ausgebildet, einen jeweiligen Morphologie-Vektor mit wenigstens einem gespeicherten Referenz-Vektor zu vergleichen, indem die Morphologie-Diskriminierungseinheit einen Abstand zwischen dem jeweiligen Morphologie-Vektor und dem Referenzvektor bestimmt, sowie einen jeweiligen Morphologie-Vektor in Abhängigkeit des Abstandes zum Referenz-Vektor einer supraventrikulären Tachykardie oder einer ventrikulären Tachykardie zuzuordnen.

Alternativ oder vorzugsweise zusätzlich ist die Morphologieerfassungseinheit ausgebildet,

- innerhalb eines Signalabschnitte die Amplituden des absoluten Signalmaximums und das absoluten Signalminimums zu detektieren und aus deren Differenz einen Spitzen-Spitzen-Wert dritter Art zu bestimmen, und

- einen zu einer von dem Signalabschnitt und der zugehörigen Nulllinie eingeschlossenen absoluten Flächengröße proportionalen Flächenwert zu bestimmen und diesen Flächenwert auf den Spitzen-Spitzen-Wert dritter Art zu normieren und so einen normierten Flächenwert zu bilden.

Die Morphologieerfassungseinheit bestimmt also die zwischen jeweils zwei Nulldurchgängen des Signals in dem Signalabschnitt liegenden Signalmaxima bzw. -minima, wobei unter einem Signalminimum eine jeweils größte Spitzenamplitude mit negativem Vorzeichen zu verstehen ist.

Mit absoluter Flächengröße ist die Flächengröße gemeint, die sich zwischen der Nulllinie und einem gleichgerichteten Signal innerhalb des Signalabschnitts ergibt, also eine Flächengröße ohne Berücksichtigung negativer Vorzeichen.

In diesem Fall ist die Morphologie-Diskriminierungseinheit ausgebildet, einen jeweiligen normierten Flächenwert mit wenigstens einem gespeicherten Referenz-Flächenwert zu vergleichen und je nach dem, wie groß der Unterschied zwischen dem jeweiligen normierten Flächenwert und dem gespeicherten Referenz-Flächenwert ist, den jeweiligen normierten Flächenwert einer supraventrikulären Tachykardie oder einer ventrikulären Tachykardie zuzuordnen.

Neben der möglichen hohen Erkennungsquote bietet die efindungsgemäße Signalverarbeitungsvorrichtung den Vorteil, dass sie bisherige Methoden zur VT/SVT-Diskrimierung sinnvoll ergänzt und vorzugsweise mit solchen, z.B. auf der Intervallauswertung basierenden Methoden zu kombinieren ist. In Verbindung mit rhythmologischen Verfahren sind mit der Morphologie-Auswertung bessere Klassifikationsergebnisse als bei den vorbekannten Lösungen zu erwarten. Dadurch, dass Fehldetektionen verringert werden, wird die Funktion des Gerätes verbessert und damit die Lebensqualität der Patienten gesteigert.

Vorzugsweise ist die Signalverarbeitungsvorrichtung ausgebildet, einen Morphologie-Vektor als Referenz-Vektor oder alternativ oder zusätzlich einen Flächenwert als Flächen-referenzwert selbsttätig zu speichern, wenn als zumindest eine Bedingung erfüllt ist, dass die Signalverarbeitungsvorrichtung erfasst hat, dass der dem jeweiligen Morphologie-Vektor zugeordnete Signalabschnitt ein Abschnitt eines Elektrokardiogramms bei Vorliegen eines normalen Sinus-Rhythmus ist. Die Signalverarbeitungsvorrichtung erkennt in diesem Fall beispielsweise anhand rhythmologischer Kennwerte wie der Herzrate und/oder der Herzratenstabilität das Vorliegen eines stabilen Sinus-Rhythmus und speichert zumindest einen in dieser Zeit generierten Morphologie-Vektor und/oder Flächenwert als einem Sinus-Rhythmus zugeordneten Referenz-Vektor bzw. Referenzflächenwert für einen späteren Vergleich mit jeweils aktuellen Morphologie-Vektoren und/oder Flächenwerten. Auf diese Weise kann die Signalverarbeitungsvorrichtung immer auf aktuelle, patientenindividuelle Referenz-Vektoren bzw. Referenzflächenwerte zurückgreifen.

In Bezug auf eine Auswertung der jeweils aktuellen Morphologie-Vektoren ist eine Signalverarbeitungsvorrichtung bevorzugt, bei der die Morphologie-Diskriminierungseinheit ausgebildet ist, den euklidischen Abstand zwischen dem jeweiligen Morphologie-Vektor und dem Referenzvektor zu bestimmen.

In Bezug auf eine Auswertung der jeweils aktuellen Flächenwerte ist eine Signalverarbeitungsvorrichtung bevorzugt, bei der die Morphologie-Diskriminierungseinheit ausgebildet ist, den Unterschied zwischen dem jeweiligen normierten Flächenwert und dem gespeicherten Referenz-Flächenwert aus der durch den Referenzwert geteilten absoluten Differenz des aktuellen Flächenwertes zum Referenzwert zu bestimmen.

Vorzugweise ist die Signalverarbeitungsvorrichtung außerdem dazu ausgebildet, für eine SVT/VT Diskriminierung sowohl einen jeweiligen Flächenwert als auch einen jeweiligen Morphologie-Vektor heranzuziehen, indem die Signalverarbeitungsvorrichtung ausgebildet ist, aus dem Abstand zwischen dem jeweiligen Morphologie-Vektor und dem Referenz-vektor sowie dem Unterschied zwischen dem jeweiligen normierten Flächenwert und dem gespeicherten Referenz-Flächenwert einen morphologischen Abstand zu ermitteln und diesen morphologischen Abstand mit einer Entscheider-Schwelle zu vergleichen. Die Morphologie-Diskriminierungseinheit ist dann vorzugsweise dazu ausgebildet, einen Signalabschnitt einer ventrikulären Tachykardie zuzuordnen, wenn der morphologische Abstand zu dem einem Sinus-Rhythmus zugeordneten Referenz-Vektor und der Referenzfläche größer ist, als der jeweilige Wert der Entscheider-Schwelle.

Besonders bevorzugt ist eine Signalverarbeitungsvorrichtung, die dazu ausgebildet ist, die Entscheider-Schwelle im Betrieb automatisch nachzuführen.

Die SVTNT Diskriminierung kann weiter verbessert werden, wenn die Signalverarbeitungsvorrichtung ausgebildet ist, neben einer Signalmorphologie zusätzlich einen, einen jeweiligen Herzrhythmus charakterisierende rhythmologischen Kennwert auszuwerten. Vorzugsweise ist die Signalverarbeitungseinrichtung Bestandteil eines implantierbaren Kardioverter/Defibrillators (ICD) und zwar insbesondere eines Einkammer-Kardioverter/Defibrillators. Die Signalverarbeitungsvorrichtung bietet nämlich den Vorteil, hierfür geeignet zu sein, weil sie eine VT/SVT-Diskriminierung allein auf Basis von in einem Ventrikel aufzunehmenden Signalwerten durchführen kann.

Statt jeweils nur einen Referenzwert, also einen Referenz-Vektor und/oder einen Flächen-referenzwert vorzusehen, die z.B. jeweils für einen Sinus-Rhythmus charakteristisch sind, können auch weitere Referenz-Vektoren und/oder Flächenreferenzwerte vorgesehen werden. Diese können zum Beispiel Werte enthalten, wie sie für ventrikuläre Tachykardien typisch sind. Die Signalverarbeitungsvorrichtung ist dann vorzugsweise dazu ausgebildet, einen ersten Vergleich mit den für einen Sinus-Rhythmus charakteristischen Referenzwerten und einen zweiten Vergleich mit den für eine ventrikuläre Tachykardie charakteristischen Referenzwerten durchzuführen und eine Zuordnung (VT/SVT Diskriminierung) unter Berücksichtigung beider Vergleichsergebnisse durchzuführen.

Die Erfindung soll nun anhand von Ausführungsbeispielen mit Bezug auf die Figuren näher erläutert werden. Von den Figuren zeigen:

Fig. 1: einen Einkammer-Kardioverter/Defibrillator mit angeschlossener Elektrodenleitung;

Fig. 2: einige Komponenten des Einkammer-Kardioverter/Defibrillators aus Fig. 1 in einem schematischen Blockschaltbild;

Fig.3: einen Dreikammer-Kardioverter/Defibrillator mit angeschlossenen Elektrodenleitungen;

Fig. 4: einige Komponenten des Dreikammer-Kardioverter/Defibrillators aus Figur 3 in einem schematischen Blockschaltbild;

Fig. 5: beispielhafte intrakardiale Elektrokardiogramme im Falle einer Erregung des Ventrikels über den AV-Knoten, wie sie auch im Falle einer supraventrikulären Tachykardie auftritt (in der Fig. 5 mit "Sinus" bezeichnet), sowie im Falle einer ventrikulären Tachykardie (in Figur 5 mit "VT" bezeichnet")

Fig. 6: erläutert eine Morphologieanalyse auf Basis der Methode der Zackendiffe- renzen;

Fig. 7: erläutert eine Morphologieanalyse auf Basis der Methode der normierten Gesamtfläche des QRS-Komplexes;

Fig. 8: einen beispielhaften Ablauf einer Klassifikation durch den Vergleich des morphologischen Abstands mit einer Entscheider-Schwelle;

Fig. 9: ein Ablaufdiagramm zur Illustration der Bestimmung eines jeweiligen Mor- phologie-Vektors;

Fig. 10: ein Ablaufdiagramm zur Illustration einer bevorzugten Bestimmung des morphologischen Abstandes eines Morphologie-Vektors eines QRS- Komplexes zu einem Referenzvektor; und

Fig. 11: ein Ablaufdiagramm zur Illustration der VT/SVT-Diskriminierung anhand des morphologischen Abstands und einer Entscheider-Schwelle.

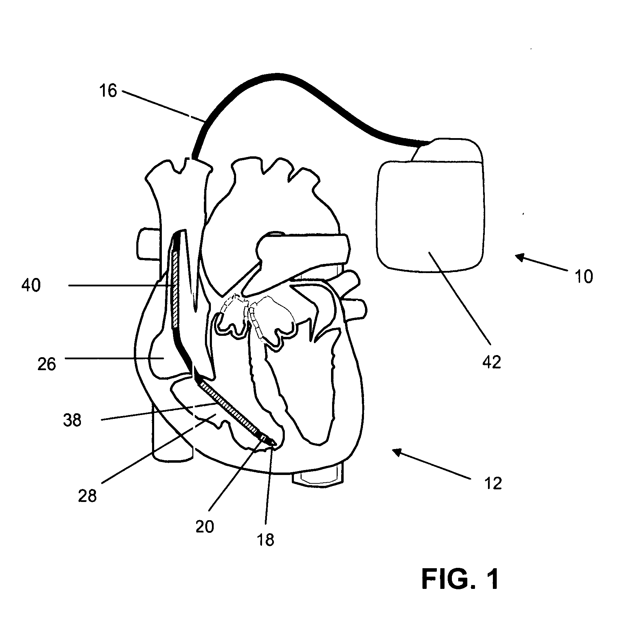

Figur 1 zeigt ein Implantat 10 in Form eines Einkammer-Herzschrittmachers und Kardioverters/Defibrillators (ICD). An diesen ist eine einzige Elektrodenleitung angeschlossen, nämlich eine rechtsventrikuläre Elektrodenleitung 16. Im implantierten Zustand endet die rechtsventrikuläre Elektrodenleitung 16 im rechten Ventrikel 28 des Herzens 12.

Die rechtsventrikuläre Elektrodenleitung 16 trägt an ihrem distalen Ende eine rechtsventrikuläre Spitzenelektrode 18 und wenig entfernt davon eine rechtsventrikuläre Ringelektrode 20. Diese Elektroden dienen der Aufnahme elektrischer Potenziale im rechten Ventrikel sowie der Abgabe von Stimulationsimpulsen an den rechten Ventrikel im normalen Schrittmacherbetrieb.

Die rechtsventrikuläre Elektrodenleitung 16 trägt außerdem noch eine distale Schockelektrode RV 38 und eine proximale Schockelektrode SVC Coil 40, die der Abgabe von Defibrillationsschocks im Falle ventrikulären Kammerflimmerns dienen, also im Falle einer (echten) ventrikulären Fibrillation. Die distale Schockelektrode 38 ist im rechten Ventrikel angeordnet und die proximale Schockelektrode 40 in der Superior Vena Cave (SVC).

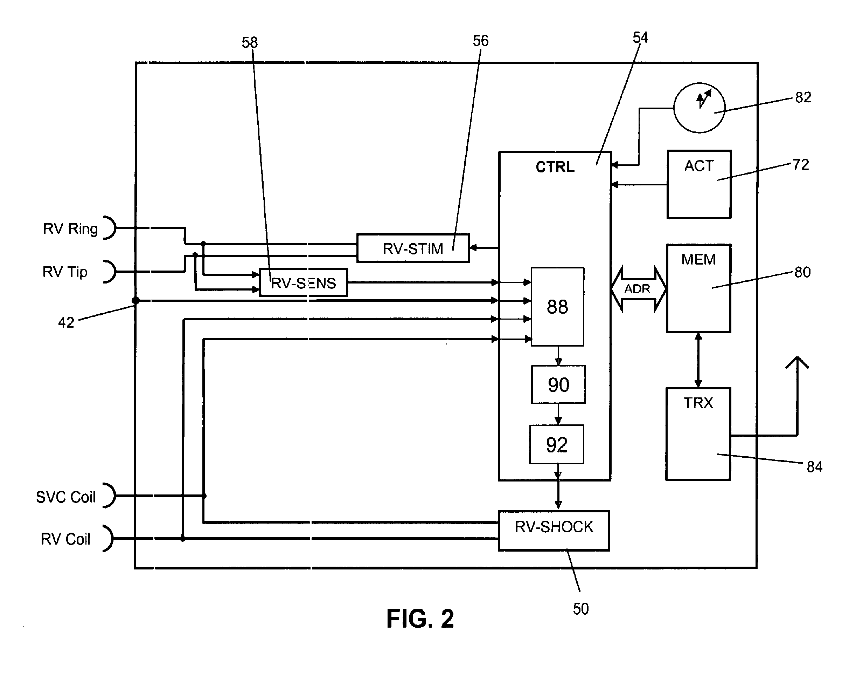

Figur 2 zeigt die Hauptbestandteile des Herzstimulators 10 aus Figur 1. Auf der linken Seite sind die elektrischen Anschlüsse für die verschiedenen Elektroden 18, 20, 38 und 40 dargestellt. Die Schockelektroden 38 und 40 sind mit einem rechtsventrikulären Schockimpulsgenerator 50 verbunden. Der Schockgenerator 50 ist mit einer Stimulationssteuereinheit 54 verbunden, die den Schockimpulsgenerator 50 bei Bedarf zur Erzeugung und Abgabe eines Defibrillationsschocks ansteuert. Weiterhin umfasst der Schockgenerator 50 in einer bevorzugten Ausführungsform in Fig. 2 nicht näher dargestellte Umschaltmittel, mittels derer entweder eine oder beide der Schockelektroden 38 und 40 und/ oder das Gehäuse 42 mit dem Schockgenerator verbunden sind, so dass der Defibrillationsschock über eine beliebige Kombination der Schockelektroden 38 und 40 und des Gehäuses 42 abgegeben werden kann..

Der Anschluss für die rechtsventrikuläre Spitzenelektrode RV Tip sowie der Anschluss für die rechtsventrikuläre Ringelektrode RV Ring sind jeweils sowohl mit einer rechtsventrikulären Stimulationseinheit 56 als auch mit einer rechtsventrikulären Sensing-Einheit 58 verbunden. Sowohl die rechtsventrikuläre Stimulationseinheit 56 als auch die rechtsventrikuläre Sensing-Einheit 58 sind jeweils mit der Stimulationssteuereinheit 54 verbunden.

Die rechtsventrikuläre Stimulationseinheit 56 ist dazu ausgebildet, auf ein Ansteuersignal der Stimulationssteuereinheit 54 hin einen rechtsventrikulären Stimulationsimpuls zu erzeugen und im Anschluss für die rechtsventrikuläre Ringelektrode RV Ring die rechtsventrikuläre Spitzenelektrode RV Tip abzugeben. Alternativ ist es auch möglich, dass das Gehäuse 42 des Herzstimulators 10 eine neutrale Elektrode bildet und die rechtsventrikuläre Stimulationseinheit 56 mit dem Anschluss für die rechtsventrikuläre Ringelektrode RV Tip und dem Gehäuse 42 als andere Elektrode zur Abgabe eines Stimulationsimpulses verbunden ist. Ein rechtsventrikulärer Stimulationsimpuls unterscheidet sich von einem Defibrillationsschock dadurch, dass der Stimulationsimpuls eine wesentlich geringere Impulsstärke besitzt, so dass er nicht wie ein Defibrillationsschock auf einen Schlag das vollständige Herzgewebe (Myokard) einer Herzkammer erregt, sondern nur die Herzmuskelzellen in unmittelbarer Umgebung der rechtsventrikulären Spitzenelektrode RV Tip 18. Diese Erregung breitet sich dann durch natürliche Reizleitung über den gesamten rechten Ventrikel 28 weiter aus und sorgt so für eine stimulierte Kontraktion des rechten Ventrikels 28.

Die rechtsventrikuläre Sensing-Einheit 58 ist dazu ausgebildet, an dem Anschluss für die rechtsventrikuläre Ringelektrode RV Ring und die rechtsventrikuläre Tippelektrode RV Tip anliegende elektrische Potentiale zunächst durch einen Eingangsverstärker zu verstärken und zu filtern. Weiterhin ist die rechtsventrikuläre Sensing-Einheit 58 ausgebildet, den Verlauf der an ihren Eingängen anliegenden elektrischen Signale derart auszuwerten, dass die rechtsventrikuläre Sensing-Einheit 58 selbsttätig eine natürliche (intrinsische), d.h. selbsttätige, Kontraktion des rechten Ventrikels 28 detektiert. Dies kann beispielsweise dadurch geschehen, dass der Verlauf des an den Eingängen der rechtsventrikulären Sensing-Einheit 58 anliegenden Signals mit einem Schwellwert verglichen wird. Typischerweise ist die größte Amplitude des Signals in Form der sogenannten R-Zacke kennzeichnend für eine natürliche Kontraktion des rechten Ventrikels 28, die durch Schwellwertvergleich detektiert werden kann. Die rechtsventrikuläre Sensing-Einheit 58 gibt daraufhin ein entsprechendes, eine natürliche Kontraktion des rechten Ventrikels 28 anzeigendes Ausgangssignal an die Stimulationssteuereinheit 54 aus.

In gleicher Art und Weise können auch für die Schockelektroden 38 und 40 eine oder mehrere (in Figur 2 nicht dargestelle) Sensing-Einheiten vorgesehen sein. Diese Sensing-Einheit oder Sensing-Einheiten sind bevorzugt ausgebildet, Signale zwischen den Schockelektroden 38 und 40, zwischen den Schockelektrode 38 und Gehäuse 42 oder zwischen Schockelektrode 40 und Gehäuse 42 zu erfassen. Dies ist in Fig. 2 schematisch durch die Verbindung der Schockelektroden 38 und 40 sowie des Gehäuses 42 mit der Elektrokardiogramm- (EKG-) Erfassungseinheit 88 dargestellt.

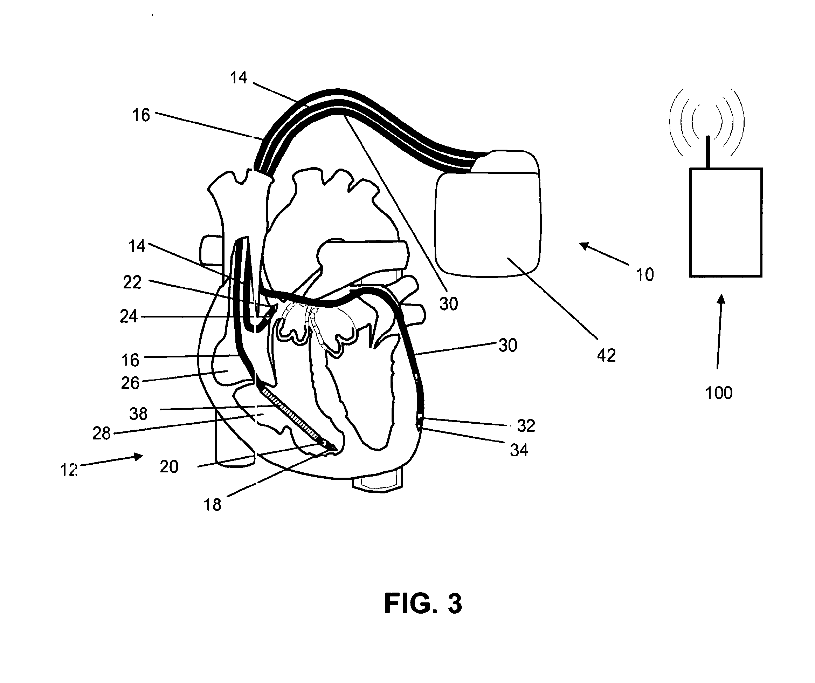

Fig. 3 zeigt ein Implantat 10 in Form eines biventrikulären Dreikammer-Herzschrittmachers und Kardioverters/ Defibrillators (ICD). An diesen sind drei Elektrodenleitungen angeschlossen, nämlich eine rechtsatriale Elektrodenleitung 14, eine rechtsventrikuläre Elektrodenleitung 16 und eine linksventrikuläre Elektrodenleitung 30. Im implantierten Zustand endet die rechtsatriale Elektrodenleitung 14 im rechten Atrium 26 eines Herzens 12. Die rechtsventrikuläre Elektrodenleitung 16 endet im rechten Ventrikel 28 des Herzens 12 und die linksventrikuläre Elektrodenleitung 30 reicht über den Coronarsinus des Herzens 12 bis zum linken Ventrikel des Herzens.

Die rechtsatriale Elektrodenleitung 14 trägt an ihrem distalen Ende eine rechtsatriale Spitzenelektrode 22 und in geringem Abstand davon eine rechtsatriale Ringelektrode 24. In ähnlicher Weise trägt die rechtsventrikuläre Elektrodenleitung 16 an ihrem distalen Ende eine rechtsventrikuläre Spitzenelektrode 18 und wenig entfernt davon eine rechtsventrikuläre Ringelektrode 20. Auch am distalen Ende der linksventrikulären Elektrodenleitung 30 ist eine linksventrikuläre Spitzenelektrode 34 und wenig entfernt davon eine linksventrikuläre Ringelektrode 32 angebracht. Diese Elektroden dienen der Aufnahme elektrischer Potenziale in der jeweiligen Herzkammer sowie der Abgabe von Stimulationsimpulsen an die jeweilige Herzkammer im normalen Schrittmacherbetrieb. Diese braucht an dieser Stelle nicht mehr erläutert zu werden.

Die rechtsventrikuläre Elektrodenleitung 16 trägt außerdem noch eine im implantierten Zustand im rechten Ventrikel angeordnete rechtsventrikuläre Schockwendel 38 als Defibrillationselektrode.

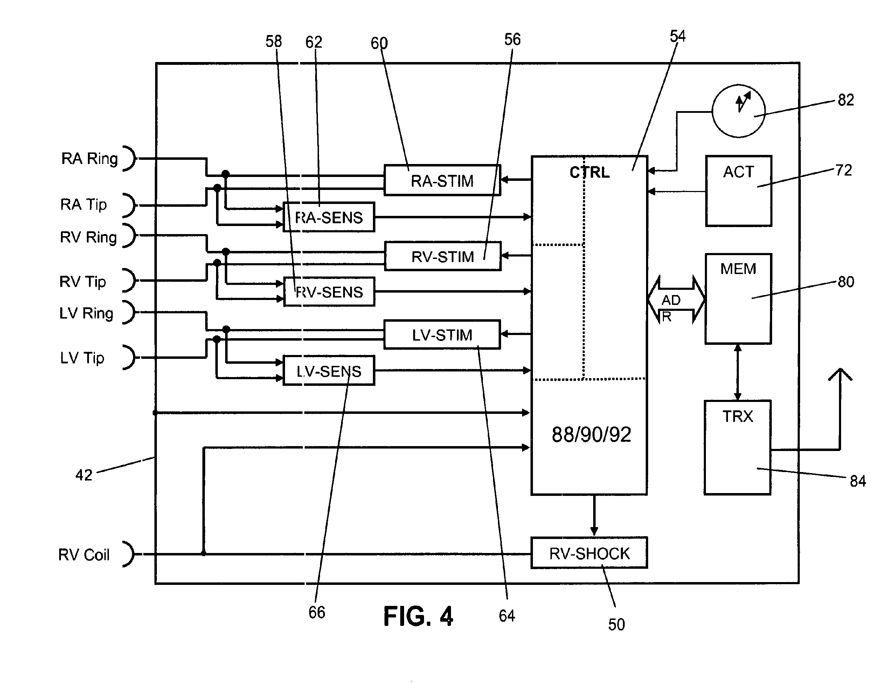

In Figur 4 sind die Hauptbestandteile des Herzstimulators 10 dargestellt. Ähnlich Figur 2 sind auf der linken Seite die elektrischen Anschlüsse für die verschiedenen Elektroden 18, 20, 22, 24, 32, 34 und 38 dargestellt. Die (einzige) Schockelektrode 38 ist mit einem rechtsventrikulären Schockimpulsgenerator 50 verbunden.

In Bezug auf die rechtsventrikuläre Spitzenelektrode RV Tip sowie die rechtsventrikuläre Ringelektrode RV Ring, das Gehäuse 42 (als Neutralelektrode), die rechtsventrikuläre Stimulationseinheit 56, die rechtsventrikuläre Sensing-Einheit 58 und die Stimulationssteuereinheit 54 gilt entsprechend das oben zu Figur 2 Gesagte.

In analoger Weise sind der Anschluss für die rechtsatriale Spitzenelektrode RA Tip und der Anschluss für die rechtsatriale Ringelektrode RA Ring sowohl mit einer rechtsatrialen Stimulationseinheit 60 als auch mit einer rechtsatrialen Sensing-Einheit 62 verbunden, die jeweils ihrerseits wiederum mit der Stimulationssteuereinheit 54 verbunden sind. Die rechtsatriale Stimulationseinheit 60 ist dazu ausgebildet, Stimulationsimpulse zu erzeugen, deren Stärke ausreicht, das rechtsatriale Myokard zu erregen. Die rechtsatrialen Stimulationsimpulse können dabei eine andere Impulsstärke besitzen als die rechtsventrikulären Stimulationsimpulse. Die rechtsatriale Sensing-Einheit 62 ist ausgebildet, aus dem Verlauf des an ihren Eingängen anliegenden Differenzsignals eine sogenannte P-Welle zu detektieren, die eine natürliche (intrinsische) Kontraktion des rechten Atriums 26 kennzeichnet. Detektiert die rechtsatriale Sensing-Einheit 62 eine entsprechende P-Welle, erzeugt sie ein Ausgangssignal und gibt dieses an die Stimulationssteuereinheit 54 weiter, welches eine natürliche Kontraktion des rechten Atriums 26 kennzeichnet.

In gleicher Weise sind auch der Anschluss für die linksventrikuläre Spitzenelektrode LV Tip und der Anschluss für die linksventrikuläre Ringelektrode LV Ring mit einer linksventrikulären Stimulationseinheit 64 und einer linksventrikulären Sensing-Einheit 66 verbunden. Die linksventrikuläre Stimulationseinheit 64 und die linksventrikuläre Sensingeinheit 66 sind ebenso mit der Stimulationssteuereinheit 54 verbunden. Beide funktionieren ähnlich wie die bereits beschriebenen Stimulationseinheiten 56 und 60 und Sensingeinheiten 58 und 62.

In gleicher Art und Weise kann auch für die Schockelektrode 38 eine (in Figur 4 nicht dargestelle) Sensing-Einheit vorgesehen sein. Diese Sensing-Einheit ist bevorzugt ausgebildet, Signale zwischen Schockelektrode 38 und Gehäuse 42 zu erfassen. Dies ist in Figur 4 schematisch durch die Verbindung der Schockelektrode 38 sowie des Gehäuses 42 mit der EKG Erfassungseinheit 88 dargestellt.

Als weiterer Bestandteil des Herzstimulators 10 ist ein Beschleunigungssensor 72 mit der Stimulationssteuereinheit 54 verbunden und in das Gehäuse 42 des Herzstimulators 10 integriert. Der Beschleunigungssensor 72 ist dazu ausgebildet, ein von der körperlichen Aktivität eines Patienten abhängiges Bewegungssignal zu erfassen und ein entsprechendes, die körperliche Aktivität des Patienten anzeigendes erstes Akzelerometer-Ausgangssignal an die Stimulationssteuereinheit 54 auszugeben. Dies erlaubt es, dass die Stimulationssteuereinheit 54 das Timing der Stimulationsimpulse an den Bedarf des Patienten (hämodynamischen Bedarf) anpasst.

Weiterhin umfasst der Herzstimulator 10 eine Speichereinheit 80, die mit der Stimulationssteuereinheit 54 verbunden ist und es erlaubt, von der Stimulationssteuereinheit 54 erzeugte oder ausgewertete Signale zu speichern. Andererseits erlaubt es die Speichereinheit 80, Steuerprogramme für die Stimulationssteuereinheit 54 in veränderbarer Form zu speichern. Des Weiteren ist die Stimulationssteuereinheit 54 mit einem Zeitgeber 82 verbunden.

Die Speichereinheit 80 ist mit einer Telemetrieeinheit 84 verbunden, die es erlaubt, in der Speichereinheit 80 abgelegte Daten drahtlos an das externe Gerät 100 zu übertragen oder Programmierbefehle seitens des externen Geräts 100 zu dem Herzstimulator 10 zu übertragen und in der Speichereinheit 80 zu speichern.

Zur Aufnahme intrakardialer Elektrokardiogramme weist die Steuereinheit 54 eine EKG-Erfassungseinheit 88 auf, die einerseits mit dem Gehäuse 42 des Herzstimulators 10 als eine erste Elektrode und außerdem mit der ventrikulären Schockelektrode 38 als zweite Elektrode verbunden ist. Die Elektrokardiogramm-Erfassungseinheit 88 ist außerdem mit der Sensing-Einheit 58 verbunden. Die Sensing-Einheit 58 generiert immer dann, wenn sie ein ventrikuläres Ereignis erfasst (siehe oben), ein ventrikuläres Markersignal. Die Elektrodkardiogramm-Erfassungseinheit 88 nutzt dieses Markersignal, um in seinem jeweils erfassten Elektrodkardiogramm einen Signalabschnitt zu detektieren, der einen QRS-Komplex enthält. Der jeweils von der Elektrokardiogramm-Erfassungseinheit 88 erfasste Signalabschnitt des intrakardialen Elektrokardiogramms ist ein Signalabschnitt in einem Zeitfenster um einen von der ventrikulären Sensing-Einheit 58 erfassten QRS-Komplex herum. Gegebenenfalls ist dieses Zeitfenster in seiner Zeitdauer abhängig von einer jeweils erfassten Herzrate.

Die Steuereinheit 54 ist dazu ausgebildet, auch bei einem Einkammer Kardioverter/Defibrillator, wie er in Figuren 1 und 2 abgebildet ist, eine zuverlässige Unterscheidung von supraventrikulären Tachykardien (SVT) und ventrikulären (VT) durchzuführen und nur, wenn es sich bei letzteren um ventrikuläre Fibrillationen (VF) handelt, die Abgabe eines ventrikulären Defibrillationsschocks über den Schockimpulsgenerator 50 und die daran angeschlossenen Schockelektroden 38 und/oder 40 auszulösen.

Dazu weist die Steuereinheit eine Morphologie-Erfassungseinheit 90 auf, die zum einen eine erste Signalanalyseeinheit als Spitzenwertdetektor aufweist, die ausgebildet ist, in einem Signalabschnitt des intrakardialen EKGs, der einen QRS-Komplex aufweist, globale Maxima und Minima des Signals zu detektieren und ausgehend hiervon vor allem eine absolut größte Signalamplitude und die der absolut größten Signalamplitude benachbarte größte Signalamplitude umgekehrter Polarität zu bestimmen. Zu diesen beiden Spitzenamplituden bildet die Morphologie-Erfassungseinheit den die jeweilige Amplitudengröße kennzeichnenden Amplitudenwert und hieraus wiederum den Spitzen-Spitzen-Wert erster Art des jeweiligen Signalabschnitts. Weiterhin bestimmt die Morphologie-Erfassungseinheit Spitzen-Spitzen-Werte zweiter Art als Differenz der Absolutwerte benachbarter Spitzenwerte gleicher Polarität und einen Spitzen-Spitzen-Wert dritter Art als Differenz der Amplituden des absoluten Signalmaximums und das absoluten Signalminimums. Eine zweite Signalanalyseeinheit der Morphologie-Erfassungseinheit ist dazu ausgebildet, einen Flächenwert zu bestimmen, der der Flächengröße einer von dem EKG-Signalabschnitt und der zugehörigen Null-Linie eingeschlossenen Fläche entspricht. Weiterhin ist die Morphologie-Erfassungseinheit dazu ausgebildet, wenigstens einige Spitzen-Spitzen-Werte des Signalabschnittes auf den größten Spitzen-Spitzen-Wert des Signalabschnitts zu normieren. Gleichermaßen ist die Morphologie-Erfassungseinheit dazu ausgebildet, auch den von der zweiten Signalanalyseeinheit gebildeten Flächenwert auf den zum selben Signalabschnitt des EKG-Signals gehörenden Wert der Spitzen-Spitzen-Wert dritter Art zu normieren. Die Morphologie-Erfassungseinheit bildet aus den normierten Amplitudenwerten für einen jeweiligen Signalabschnitt einen die Morphologie dieses Signalabschnitts kennzeichnenden Morphologie-Vektor, wie nachfolgend näher beschrieben ist.

Diesen Morphologie-Vektor und den auf den Spitzen-Spitzen-Wert dritter Art des jeweiligen Signalabschnitts normierten Flächenwert gibt die Morphologie-Erfassungseinheit 90 an eine Morphologie-Diskriminierungseinheit 92 aus. Die Morphologie-Diskriminierungseinheit ist ausgebildet, einen jeweiligen aktuellen Morphologie-Vektor und einen jeweils aktuellen normierten Flächenwert mit im Speicher 80 gespeicherten Referenzwerten zu vergleichen und den euklidischen Abstand zwischen einem den Morphologie-Vektor und zusätzlich den normierten Flächenwert enthaltenden Vektor und einem oder mehreren entsprechenden gespeicherten Referenz-Vektoren zu bestimmen. Auch dies ist nachfolgend näher beschrieben. Einer der gespeicherten Referenz-Vektoren ist dabei dem normalen Sinus-Rhythmus zugeordnet, während ein weiterer gespeicherter Referenzvektor einer ventrikulären Tachykardie (VT) zugeordnet sein kann.

Wenn die Berechnung des euklidischen Abstands zu einem oder mehreren Referenzvektoren ergibt, dass der aktuelle Vektor näher dem Referenzvektor ist, der dem normalen Sinus-Rhythmus zugeordnet ist, wird eine aktuelle Tachykardie als supraventrikuläre Tachykardie eingestuft, ansonsten wird die aktuelle Tachykardie als ventrikuläre Tachykardie eingestuft. Ergibt im Falle des Vergleiches des euklidschen Abstandes mit mehreren Referenzvektorendass ein aktueller Vektor einen geringeren Abstand zum eine ventrikuläre Tachykardie kennzeichnenden Referenz-Vektor hat als zu dem den normalen Sinus-Rhythmus kennzeichnenden Referenz-Vektor, wird ein jeweiliger aktueller Signalabschnitt einer ventrikulären Tachykardie zugeordnet. Im Fall der Zuordnung zu einer ventrikulären Tachykardie kann die Steuereinheit 54 gegebenenfalls unter Berücksichtigung weiterer Bedingungen die Abgabe eines Defibrillationsschocks über den Schockimpulsgenerator 50 und die daran angeschlossenen Schockelektroden auslösen.

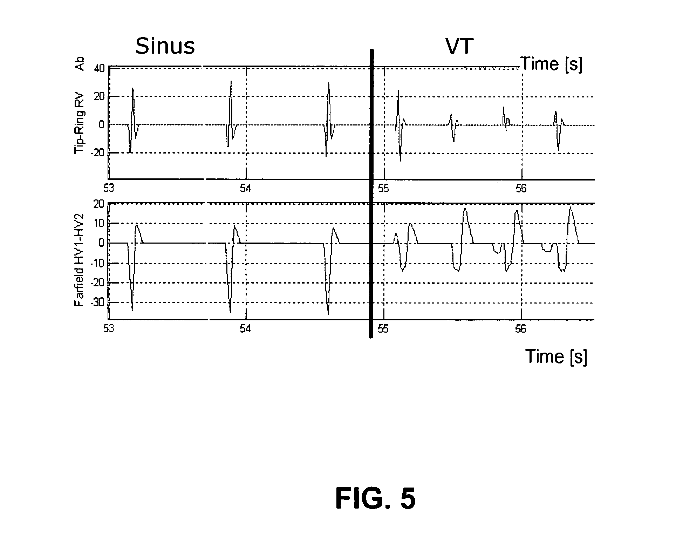

Die Morphologie-Diskriminierungseinheit beurteilt auf diese Weise die Signalform der QRS-Komplexe. Im Sinusrhythmus bleibt die Morphologie weitestgehend stabil. Bei einer supraventrikulären Tachykardie ist der Ursprung jeder Herzerregung wie beim Sinus-rhythmus im Vorhof (wenn auch nicht unbedingt im Sinusknoten) oder im AV-Knoten, so dass die Überleitung und Ausbreitung im Ventrikel meistens sehr ähnlich zu der im Sinus-rhythmus ist. Kommt es jedoch zu einer ventrikulären Tachykardie, so ist die Erregungsausbreitung im Ventrikel normalerweise sehr verschieden zu der im Sinusrhythmus, was zu einer anderen Signalmorphologie führt (siehe Figur 5). Mit Auswertung der Morphologie lassen sich also VTs von SVTs unterscheiden.

In Figur 5 sind beispielhafte intrakardiale Elektrokardiogramme im Falle einer Erregung des Ventrikels über den AV-Knoten, wie sie auch im Falle einer supraventrikulären Tachykardie auftritt (in der Figur 5 mit "Sinus" bezeichnet), sowie im Falle einer ventrikulären Tachykardie (in Figur 5 mit "VT" bezeichnet") abgebildet. Die jeweils oberen intrakardialen Elektrokardiogramme sind dabei über die rechtsventrikuläre Ringelektrode RV Ring 20 und die rechtsventrikuläre Spitzenelektrode RV Tip 18 abgeleitet, während die beiden in Figur 5 unteren intrakardialen Elektrokardiogramme über die ventrikulären Schockwendeln 38 und 40 abgeleitet sind.

Die von der Morphologieerfassungseinheit 90 und der Morphologiediskriminierungseinheit 92 gebildete Signalverarbeitungsvorrichtung, die durch die Steuereinheit 54 des Implantats 10 realisiert ist, führt vorzugsweise zwei Analysemethoden durch nämlich eine Morphologieanalyse auf Basis der Signal-Spitzamplituden (Signalzacken) - im folgenden auch als Methode der Zackendifferenzen bezeichnet - und eine Analyse auf Basis der auf einen jeweiligen Spitzen-Spitzen-Wert dritter Art genormten Fläche, die von dem Signal und der jeweiligen Nulllinie in dem Signalabschnitt eingeschlossen ist.

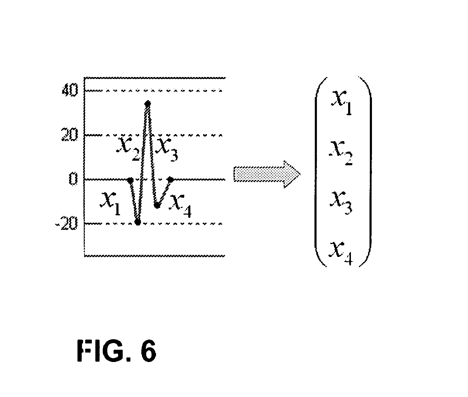

Bei der Methode der Zackendifferenzen (siehe Figur 6) werden die Signalspitzen (Spitzenamplituden) zwischen Nulldurchgängen gesucht und aus den Differenzen der Amplitudenwerte benachbarter Spitzenamplituden ein Vektor gebildet. In dem Beispiel werden die Vektorkomponenten durch die Differenz von Null und der 1. Spitzenamplitude, der 1. Spitzenamplitude und der 2. Spitzenamplitude, der 2. Spitzenamplitude und der 3. Spitzenamplitude und der 3. Spitzenamplitude und Null gebildet. Alle Amplitudendifferenz-Werte werden auf die maximale Amplitudendifferenz - also den größten der Vielzahl der Spitzen-Spitzen-Werte - normiert (nicht im Beispiel dargestellt). Die maximale Amplitudendifferenz wird separat gespeichert. Die in Figur 6 dargestellten Spitzen-Spitzen-Werte $X_2$ und $X_3$ sind Spitzen-Spitzen-Werte erster Art. Der Spitzen-Spitzen-Wert $X_2$ ist außerdem der Spitzen-Spitzen-Wert dritter Art, nämlich der Absolut größte Spitzen-Spitzen-Wert im dargestellten Signalabschnitt. Die Spitzen-Spitzen-Werte werden in Rahmen dieser Beschreibung auch als Zackendifferenzen bezeichnet und zu einem Vektor zusammengefasst.

Im Sinusrhythmus wird ein Referenzvektor erstellt. Bei einer einsetzenden Tachykardie können dann die Vektoren der QRS-Komplexe mit dem Referenzvektor verglichen werden. Hierfür kann z.B. der euklidische Abstand oder ein anderes Abstandsmaß verwendet werden.

Ein Vorteil der Methode der Zackendifferenzen ist, dass kleinere Amplitudenschwankungen oder Nulllinienschwankungen nur geringe Auswirkungen auf die Morphologie-Berechnung haben.

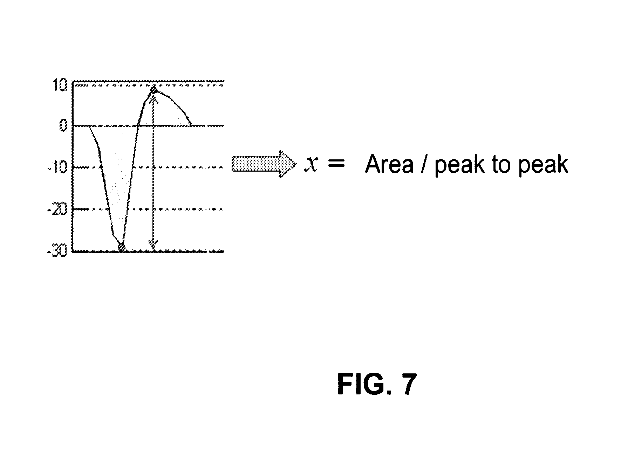

Bei der Methode der normierten Fläche (siehe Figur 7) wird ein morphologischer Wert berechnet, indem die Gesamtfläche des QRS-Komplexes durch die maximale Amplitudendifferenz, also den Spitzen-Spitzen-Wert dritter Art geteilt wird.

Ein Vorteil der Methode der normierten Fläche ist, dass Streuungen einzelner Abtastwerte kaum ins Gewicht fallen, da alle Abtastwerte eines QRS-Komplexes in die Berechnung einfließen. Nulllinienschwankungen haben kaum Auswirkung auf Fläche und den Spitzen-Spitzen-Wert.

Die Methode der normierten Fläche ist vorzugsweise mit der Methode der Zackendifferenzen verknüpft, um ein besseres Klassifikationsergebnis als mit einer einzelnen Methode zu erzielen. Es wird für jede Methode eine eigene Referenz gebildet. Die mit beiden Methoden bestimmten, jeweiligen normierten Abstandsmaße zum jeweiligen Referenzwert (Referenzvektor und Referenzflächenwert) werden zu einem gemeinsamen "morphologischen Abstand" gemittelt.

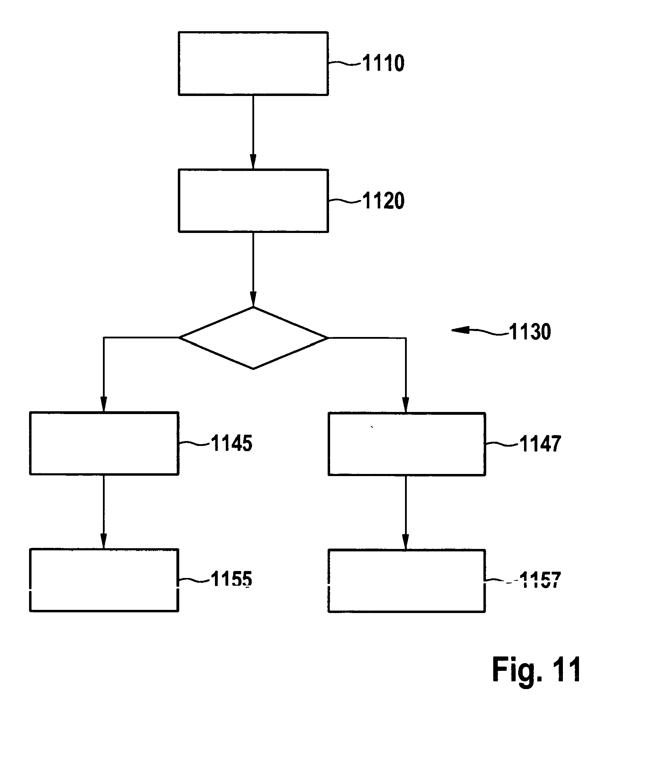

Anhand einer Entscheider-Schwelle kann nun eine Klassifikation erfolgen. Liegt der morphologische Abstand unter der Schwelle, so liegt eine Ähnlichkeit zur Referenz vor. Werte oberhalb der Schwelle weisen auf einen anderen Signalursprung hin. Dies ist in Figur 11 anhand eines Ablaufdiagramms im Detail dargestellt.

Bei der Bestimmung der Referenzwerte, die einen Sinusrhythmus kennzeichnen, werden im Sinusrhythmus für jeden QRS-Komplex (also für jedes ventrikuläre Sense-Event) die morphologischen Werte - also Morphologievektor und normierte Flächengröße - berechnet. Hierfür wird aus dem z.B. über die Schockwendel 38 und das Gehäuse 42 abgeleiteten intrakardialen Elektrokardiogramm (IEGM) ein jeweils einen QRS-Komplex enthaltender Signalabschnitt herausgeschnitten und die morphologischen Merkmale werden extrahiert. Es werden die Amplituden-Differenzen der Zacken berechnet, auf die absolut maximale Differenz normiert und in einen Vektor geschrieben. Die Vektorlänge wird z.B. auf 4 festgelegt. Außerdem wird die absolute Fläche des QRS-Komplexes berechnet und auf die peak-to-peak Amplitude (Spitzen-Spitzen-Wert dritter Art) normiert. Diese beiden Referenz-Werte werden gespeichert.

Bei der Referenzberechnung sind unterschiedliche Verfahren möglich, z.B. eine ständige oder eine intervallgesteuerte (z.B. alle 12h) Nachführung im Sinusrhythmus. Der Sinus-rhythmus kann z.B. mit einer Frequenzbedingung bestimmt werden.

Bei Auftreten einer Tachykardie werden die Zackendifferenzen und die normierte Fläche jedes QRS-Komplexes mit ihrer jeweiligen Referenz verglichen und daraus wird der "morphologische Abstand" berechnet. Anhand einer patienten-individuellen dynamischen Entscheider-Schwelle erfolgt die Klassifikation: liegt der Abstand unter der entscheidenden Schwelle, so ist die Ähnlichkeit zur Referenz groß, und es wird auf SVT entschieden. Liegt der Abstand über der entscheidenden Schwelle, so ist die Ähnlichkeit gering und es wird auf VT entschieden.

Die Entscheidung des Morphologie-Algorithmus kann nun mit anderen Kriterien des ICDs verknüpft werden, so dass die Therapie entweder unterdrückt (SVT) oder freigegeben (VT) wird.

Der Vergleich zum jeweiligen Referenzvektor erfolgt bei der Methode der Zackendifferenzen z.B. durch die Berechnung des euklidischen Vektorabstands:

$$\begin{pmatrix} x_{ref\,1} \\ x_{ref\,2} \\ x_{ref\,3} \\ x_{ref\,4} \end{pmatrix} - \begin{pmatrix} x_1 \\ x_2 \\ x_3 \\ x_4 \end{pmatrix} = \vec{e} \longrightarrow \quad \text{Euklidischer Abstand} \quad d = \sqrt{\vec{e}^T \cdot \vec{e}}$$

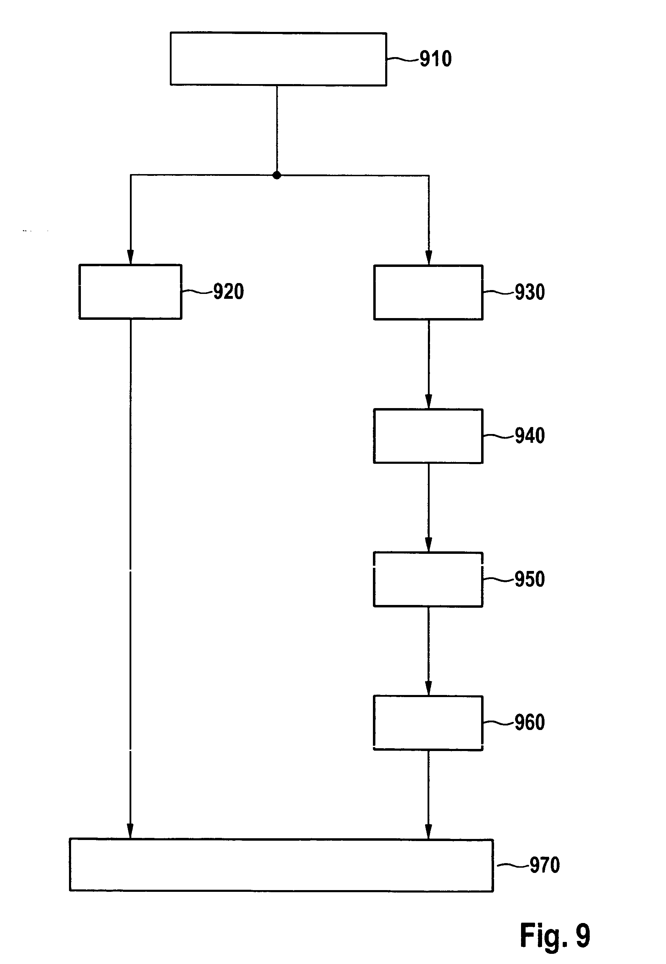

Fig. 9 zeigt ein Ablaufdiagramm zur Illustration einer bevorzugten Bestimmung eines jeweiligen Morphologie-Vektors. In einem ersten Schritt 910 bestimmt die Morphologieerfassungseinheit den aktuellen QRS-Komplex Beispielsweise entspricht der aktuelle QRS-Komplex einem Signalverlauf einer Zeitspanne von 250 ms. Danach erfolgt eine Parallelverarbeitung: Einerseits bestimmt die Morphologieerfassungseinheit in einem Schritt 920 die normierte Fläche als Quotienten des Flächenwertes und des Spitzen-Spitzen-Wertes dritter Art.

Parallel zum Schritt 920 führt die Morphologieerfassungseinheit Schritte 930, 940, 950 und 960 aus. Im Schritt 930 ermittelt die Morphologieerfassungseinheit Nulldurchgänge im QRS-Komplex, bestimmt Zacken und ermittelt Spitzen-Spitzen-Werte, die hier als Zackendifferenzen bezeichnet sind. Dieser Schritt wurde bereits im Rahmen der Beschreibung zu Fig. 6 erläutert. Im darauf folgenden Schritt 940 eliminiert die Morphologieerfassungseinheit alle Zackendifferenzen, deren betragsmäßiger Wert kleiner als der 5%ige oder gleich dem 5%igen Anteil des betragsmäßigen Wertes der maximalen Zackendifferenz sind. Im Schritt 950 normiert die Morphologieerfassungseinheit die Werte der übrigen Zackendifferenzen auf den betragsmäßigen Wert der maximalen Zackendifferenz und stellt einen mehrdimensionalen, beispielsweise vierdimensionalen Vektor auf, wobei die normierten Zackendifferenzen Skalare dieses Vektors bilden. Im Schritt 960 speichert die Morphologieerfassungseinheit zusätzlich den Wert der maximalen Zackendifferenz ab.

Im Schritt 970 übergibt die Morphologieerfassungseinheit sowohl den normierten Flächenwert als auch den mehrdimensionalen Vektor an die Morphologiediskriminierungseinheit.

Bei der Methode der normierten Fläche wird mittels normierter Differenzbildung die relative Abweichung berechnet. Die Ergebnisse der beiden Methoden werden zum morphologischen Abstand gemittelt.

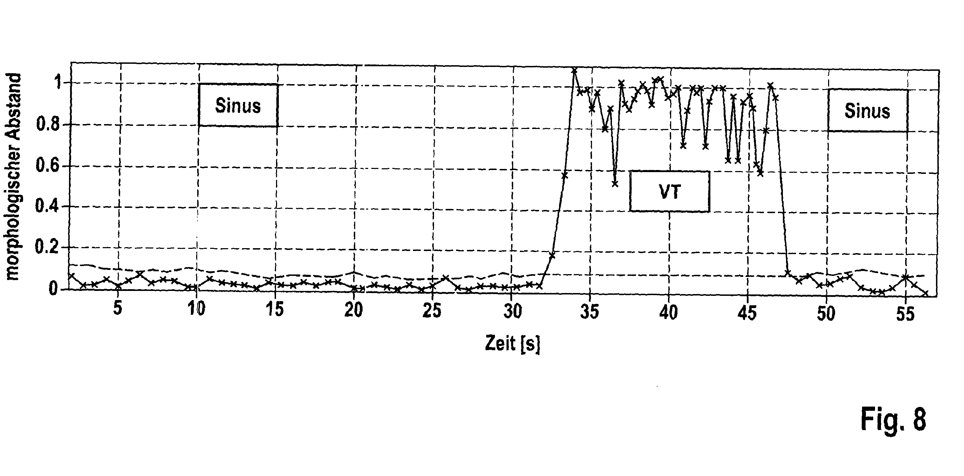

Die Klassifikation erfolgt durch den Vergleich des morphologischen Abstands mit einer Entscheider-Schwelle, wie in Figur 8 dargestellt ist.

Bei der Methode der Zackendifferenzen muss jeder Morphologie-Vektor mit dem Referenzvektor verglichen werden. Jedoch kann es vorkommen, dass die Vektoren gegeneinander verschoben sind. Es ist also zunächst eine Ausrichtung notwendig. Dafür werden aktueller Morphologie-Vektor und der jeweilige Referenzvektor gegeneinander verschoben und für jede Verschiebung der euklidische Abstand berechnet. Dazu addiert wird die relative Abweichung der maximalen Amplitudendifferenz des aktuellen Komplexes zur Referenz. Der minimale Abstand (bei den Verschiebungen) wird als Abstandswert für den aktuellen QRS-Komplex angesehen.

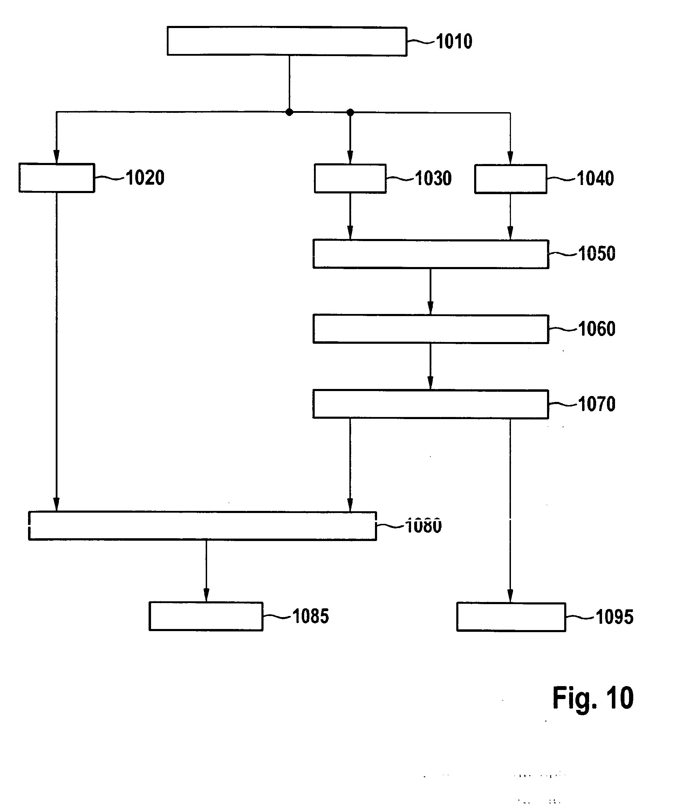

Figur 10 zeigt ein Ablaufdiagramm zur Illustration einer bevorzugten Bestimmung des morphologischen Abstandes eines Morphologievektors eines QRS-Komplexes zu einem Referenzvektor. In einem ersten Schritt 1010 übernimmt die Morphologie-Diskriminierungseinheit Werte der Morphologieerfassungseinheit. In den Schritten 1020, 1030 und 1040 nimmt die Morphologie-Diskriminierungseinheit eine Anpassung vor: Im Schritt 1020 erfolgt eine Normierung in der Weise, dass eine absolute Differenz des aktuellen normierten Flächenwertes und des normierten Flächen-Referenzwertes durch den normierten Flächen-Referenzwert geteilt wird.

Im Schritt 1030 erweitert die Morphologie-Diskriminierungseinheit den aktuellen Zackendifferenzvektor um eine oder mehrere Dimensionen, wobei im Falle eines vierdimensionalen Zackendifferenzvektors eine Erweiterung um 3 Dimensionen erfolgt. Die Skalare der hinzugefügten Dimensionen haben jeweils den Wert Null. Ebenso erweitert die Morphologie-Diskriminierungseinheit im Schritt 1040 den Referenz-Vektor auf die gleiche Anzahl Dimensionen wie der erweiterte Zackendifferenzvektor, also im Falle eines vierdimensionalen Zackendifferenzvektors auf sieben Dimensionen. Auch hier haben die Skalare der hinzugefügten Dimensionen den Wert Null.

Im Schritt 1050 berechnet die Morphologie-Diskriminierungseinheit den euklidischen Abstand bei vier Verschiebungen des aktuellen Vektors zum Referenzvektor. Dabei berücksichtigt sie im Schritt 1060 die relative Abweichung des Wertes der maximalen Zackendifferenz (Spitzen-Spitzen-Wert dritter Art) zum Referenzwert. Im Schritt 1070 bestimmt die Morphologie-Diskriminierungseinheit den Abstandswert zum Referenzvektor als minimalen euklidischen Abstand.

Diesen Abstandswert mittelt die Morphologie-Diskriminierungseinheit im Schritt 1080 mit dem im Schritt 1020 bestimmten Wert, dem normierten Flächenabstand. Diesen Mittelwert der beiden normierten Abstände gibt die Morphologie-Diskriminierungseinheit im Schritt 1085 als morphologischen Abstand aus. Zusätzlich speichert die Morphologie-Diskriminierungseinheit im Schritt 1095 eine optimale Verschiebung des aktuellen Vektors.

Bei der Methode der normierten Fläche wird der morphologische Abstand aus der absoluten Differenz des aktuellen Flächenwertes zum Referenzwert gebildet. Diese Differenz wird auf den Referenzwert normiert. Der "Abstandswert" gibt also den relativen Unterschied zum Referenzwert für den Flächenwert an:

$$Fl\ddot{a}chenabstand = \left|\left(normFl\ddot{a}che_{ref} - normFl\ddot{a}che_{aktuell}\right)\right| / normFl\ddot{a}che_{ref}$$

Als Gesamt-Abstandsergebnis eines QRS-Komplexes wird der Mittelwert des Abstandes der normierten Fläche und des Abstandes der Zackendifferenzen gebildet.

Beim euklidischen Abstand kann aus rechentechnischen Gründen auf die Wurzel verzichtet werden. Das Ergebnis entspricht dann dem quadratischen Abstand.

Für jeden Patienten wird eine individuelle dynamische Entscheider-Schwelle berechnet, die nach jedem Herzschlag automatisch angepasst wird und so im Betrieb der Signalverarbeitungsvorrichtung automatisch nachgeführt wird. Dies ist sinnvoll, da die Streuungen der intrakardialen Signale bei verschiedenen Patienten sehr unterschiedlich sein können. QRS-Komplexe, deren Abstandsmaß über der Morphologie-Schwelle liegt, werden als VT-Schläge klassifiziert, unter der Schwelle werden sie als SVT-Schläge klassifiziert.

In Figur 11 ist eine bevorzugte VT/SVT-Diskriminierung anhand des morphologischen Abstands und einer Entscheider-Schwelle illustriert. Der Algorithmus startet, sobald die Herzrate einen bestimmten Schwellwert überschritten hat. Zu Beginn werden zwei Zählvariablen auf Null gesetzt. Im Schritt 1110 erfolgen im Wesentlichen die Schritte 910 bis 970, also die Bestimmung des Morphologievektors, die bereits im Rahmen der Beschreibung zu Fig. 9 erläutert wurde. Zusätzlich wird eine erste Zählvariable um 1 erhöht.. Im Schritt 1120 erfolgen im Wesentlichen die Schritte 1010 bis 1085, also die die Bestimmung der morphologischen Abstandes, die bereits im Rahmen der Beschreibung zu Fig. 10 erläutert wurde.

Im Schritt 1130 führt die Morphologie-Diskriminierungseinheit einen Vergleich zwischen dem Wert des morphologischen Abstandes und dem Wert der Entscheider-Schwelle. Ist der Wert des morphologischen Abstandes größer als der Wert der Entscheider-Schwelle, so ordnet die Morphologie-Diskriminierungseinheit im Schritt 1145 dem aktuellen QRS-Komplex eine ventrikuläre Tachykardie zu und erhöht im Schritt 1155 den Wert einer zweiten Zählvariable um 1. Im anderen Fall, ordnet die Morphologie-Diskriminierungseinheit im Schritt 1147 dem aktuellen QRS-Komplex eine supraventrikuläre Tachykardie oder einen Sinus-Rhythmus zu und verringert den Wert der zweiten Zähl-Zählvariable um 1, allerdings nur dann, wenn der Wert der zweiten Zählvariable größer als Null ist. Solange die Herzrate einen bestimmten Schwellwert überschritten hat wird dann der Algorithmus mit beginnend mit Schritt 1110 wiederholt.

Anhand des Wertes der beiden Zählvariablen kann die Stimulationssteuereinheit die Therapie steuern. So kann beispielsweise entschieden werden, dass eine ventrikuläre Tachykardie vorliegt, wenn der Wert der zweiten Zählvariable größer ist als die Hälfte des Wertes der zweiten Zählvariable.

**Bezugszeichenliste**

10    Herzstimulator
12    Herz
14    Rechtsatriale Elektrodenleitung
16    Rechtsventrikuläre Elektrodenleitung
18    Rechtsventrikuläre Spitzenelektrode
20    Rechtsventrikuläre Ringelektrode
22    Rechtsatriale Spitzenelektrode
24    Rechtsatriale Ringelektrode
26    Rechtes Atrium
28    Rechtes Ventrikel
30    Linksventrikuläre Elektrodenleitung
32    Linksventrikuläre Ringelektrode
34    Linksventrikuläre Spitzenelektrode
38    Distale Schockelektrode
40    Proximale Schockelektrode
42    Gehäuse

| 50 | Rechtsventrikulärer Schockimpulsgenerator |
| 54 | Stimulationssteuereinheit |
| 56 | Rechtsventrikuläre Stimulationseinheit |
| 58 | Rechtsventrikuläre Sensing-Einheit |
| 60 | Rechtsventrikuläre Stimulationseinheit |
| 62 | Rechtsatriale Sensing-Einheit |
| 64 | Linksventrikuläre Stimulationseinheit |
| 66 | Linksventrikuläre Sensing-Einheit |
| 72 | Beschleunigungssensor |
| 80 | Speichereinheit |
| 82 | Zeitgeber |
| 84 | Telemetrieeinheit |
| 88 | EKG-Erfassungseinheit |
| 90 | Morphologieerfassungseinheit |
| 92 | Morphologiediskriminierungseinheit |
| 100 | Externes Gerät |

**Patentansprüche**

1. Signalverarbeitungsvorrichtung zur Klassifikation intrakardialer Signale, mit einem Signaleingang für ein physiologisches Signal, insbesondere ein Elektrokardiogramm, sowie mit einer Morphologieerfassungseinheit (90), die mit dem Signaleingang zumindest indirekt verbunden und ausgebildet ist,
Signalmaxima und -minima in einem Signalabschnitt zu detektieren und zu einer vorgegebenen Zahl von Signalmaxima und -minima jeweils einen zur Größe der Spitzenamplitude proportionalen Spitzenwert zu bestimmen,
und aus zwei benachbarten Spitzenwerten mit entgegengesetzter Polarität einen Spitzen-Spitzen-Wert erster Arzt als Summe der Absolutwerte der einzelnen Spitzenwerte zu bestimmen
und im selben Signalabschnitt weitere Spitzen-Spitzen-Werte erster Art als Summe der Absolutwerte benachbarter Spitzenwerte entgegengesetzter Polarität zu bestimmen,
und die Spitzen-Spitzen-Werte auf den größten der bestimmten Spitzen-Spitzen-Wert zu normieren und so eine vorgegebene Anzahl normierter Spitzen-Spitzen-Werte zu bilden, die zusammen einen Morphologie-Vektor ergeben, der für die Morphologie eines jeweiligen Signalabschnitts charakteristisch ist, und mit
einer Morphologie-Diskriminierungseinheit (92), die mit der Morphologieerfassungseinheit (90) verbunden und ausgebildet ist, einen jeweiligen Morphologie-Vektor mit wenigstens einem gespeicherten Referenz-Vektor zu vergleichen, indem die Morphologie-Diskriminierungseinheit (92) einen Abstand zwischen dem jeweiligen Morphologie-Vektor und dem Referenzvektor bestimmt, sowie einen jeweiligen Morphologie-Vektor in Abhängigkeit des Abstandes zum Referenz-Vektor einer supraventrikulären Tachykardie oder einer ventrikulären Tachykardie zuzuordnen.

2. Signalverarbeitungsvorrichtung nach Anspruch 1, **dadurch gekennzeichnet, dass** die Morphologieerfassungseinheit (90) ausgebildet ist,
weitere, Spitzen-Spitzen-Werte zweiter Art als Differenz der Absolutwerte benachbarter Spitzenwerte gleicher Polarität zu bestimmen.

3. Signalverarbeitungsvorrichtung nach Anspruch 1 oder 2, **dadurch gekennzeichnet, dass** die Signalverarbeitungsvorrichtung ausgebildet ist, einen Morphologie-Vektor bei Bedarf als Referenz-Vektor selbsttätig zu speichern, wenn die Signalverarbeitungsvorrichtung erfasst hat, dass der dem jeweiligen Morphologie-Vektor zugeordnete Signalabschnitt ein Abschnitt eines Elektrokardiogramms bei Vorliegen eines normalen Sinus-Rhythmus ist.

4. Signalverarbeitungsvorrichtung nach Anspruch 1 oder 2, **dadurch gekennzeichnet, dass** die Morphologie-Diskriminierungseinheit (92) ausgebildet ist, den Abstand zwischen dem jeweiligen Morphologie-Vektor und dem Referenzvektor als euklidischen Abstand zu bestimmen.

5. Signalverarbeitungsvorrichtung zur Klassifikation intrakardialer Signale, mit einem Signaleingang für ein physiologisches Signal, insbesondere ein Elektrokardiogramm, sowie mit einer Morphologieerfassungseinheit (90), die mit dem Signaleingang zumindest indirekt verbunden und ausgebildet ist,
innerhalb eines Signalabschnittes die Amplituden des absoluten Signalmaximums und des absoluten Signalminimums zu detektieren und aus deren Differenz einen Spitzen-Spitzen-Wert dritter Art zu bestimmen, und
einen zu einer von dem Signalabschnitt und der zugehörigen Nulllinie eingeschlossenen absoluten Flächengröße proportionalen Flächenwert zu bestimmen und diesen Flächenwert auf den Spitzen-Spitzen-Wert dritter Art zu normieren und so einen normierten Flächenwert zu bilden,
und mit
einer Morphologie-Diskriminierungseinheit (92), die mit der Morphologieerfassungseinheit (90) verbunden und ausgebildet ist, einen jeweiligen normierten Flächenwert mit wenigstens einem gespeicherten Referenz-Flächenwert zu vergleichen und je nach dem, wie groß der Unterschied zwischen dem jeweiligen normierten Flächenwert und dem gespeicherten Referenz-Flächenwert ist, den jeweiligen normierten Flächenwert einer supraventrikulären Tachykardie oder einer ventrikulären Tachykardie zuzuordnen.

6. Signalverarbeitungsvorrichtung nach Anspruch 5, **dadurch gekennzeichnet, dass** die Morphologie-Diskriminierungseinheit (92) ausgebildet ist, den Unterschied zwischen dem jeweiligen normierten Flächenwert und dem gespeicherten Referenz-Flächenwert aus einer durch den Referenz-Flächenwert geteilten absoluten Differenz des aktuellen Flächenwertes zum Referenz-Flächenwert zu bestimmen.

7. Signalverarbeitungsvorrichtung nach Anspruch 1 und Anspruch 5, **dadurch gekennzeichnet, dass** die Signalverarbeitungsvorrichtung ausgebildet ist, aus dem Abstand zwischen dem jeweiligen Morphologie-Vektor und dem Referenzvektor sowie dem Unterschied zwischen dem jeweiligen normierten Flächenwert und dem gespeicherten Referenz-Flächenwert einen morphologischen Abstand zu mitteln und diesen morphologischen Abstand mit einer Entscheider-Schwelle zu vergleichen.

8. Signalverarbeitungsvorrichtung nach Anspruch 7, **dadurch gekennzeichnet, dass** die Morphologie-Diskriminierungseinheit (92) ausgebildet ist, einen Signalabschnitt einer ventrikulären Tachykardie zuzuordnen, wenn der morphologische Abstand zu dem einem Sinus-Rhythmus zugeordneten Referenz-Vektor und der Referenzfläche größer ist, als der jeweilige Wert der Entscheider-Schwelle.

9. Signalverarbeitungsvorrichtung nach Anspruch 7 oder 8, **dadurch gekennzeichnet, dass** die Signalverarbeitungsvorrichtung ausgebildet ist, die Entscheider-Schwelle im Betrieb automatisch nachzuführen.

10. Signalverarbeitungsvorrichtung nach Anspruch 1 oder 5, **dadurch gekennzeichnet, dass** die Signalverarbeitungsvorrichtung ausgebildet ist, neben einer Signalmorphologie zusätzlich einen jeweiligen Herzrhythmus charakterisierenden Kennwert auszuwerten.

11. Kardioverter/Defibrillator mit einer Signalverarbeitungseinrichtung gemäß einem der Ansprüche 1 bis 10.

12. Kardioverter/Defibrillator nach Anspruch 11, **dadurch gekennzeichnet, dass** der Kardioverter/Defibrillator ein Einkammer Kardioverter/Defibrillator ist.

**Claims**

1. A signal processing device for classifying intracardiac signals, comprising a signal input for a physiological signal, in particular an electrocardiogram, and a morphology detection unit (90), which is at least indirectly connected to the signal input and designed
to detect signal maxima and minima in a signal section and to determine a peak value that is proportional to the magnitude of the peak amplitude for each of a predetermined number of signal maxima and minima; and
to determine a peak-to-peak value of a first type from two adjacent peak values having opposite polarities as a sum of the absolute values of the individual peak values; and
to determine, in the same signal section, additional peak-to-peak values of the first type as a sum of the absolute values of adjacent peak values having opposite polarities; and
to normalize the peak-to-peak values to the largest one of the peak-to-peak values that were determined and thus create a predetermined number of normalized peak-to-peak values, which together result in a morphology vector, which is characteristic of the morphology of a particular signal section, and further comprising
a morphology discrimination unit (92), which is connected to the morphology detection unit (90) and is designed to compare a respective morphology vector to at least one stored reference vector in that the morphology discrimination unit (92) identifies a distance between the respective morphology vector (92) and the reference vector, and to categorize a respective morphology vector as supraventricular tachycardia or ventricular tachycardia, depending on the distance from the reference vector.

**2.** The signal processing device according to claim 1, **characterized in that** the morphology detection unit (90) is designed
to determine additional peak-to-peak values of a second type as a difference between the absolute values of adjacent peak values having the same polarity.

**3.** The signal processing device according to claim 1 or 2, **characterized in that** the signal processing device is designed to automatically store a morphology vector, if needed, as a reference vector when the signal processing device has detected that the signal section associated with the respective morphology vector is a segment of an electrocardiogram when a normal sinus rhythm is present.

**4.** The signal processing device according to claim 1 or 2, **characterized in that** the morphology discrimination unit (92) is designed to determine the distance between the respective morphology vector and the reference vector as the Euclidian distance.

**5.** A signal processing device for classifying intracardiac signals, comprising a signal input for a physiological signal, in particular an electrocardiogram, and a morphology detection unit (90), which is at least indirectly connected to the signal input and designed
to detect the amplitudes of the absolute signal maximum and of the absolute signal minimum within a signal section, and to determine a peak-to-peak value of a third type from the difference thereof; and
to determine an area value that is proportional to an absolute area size enclosed by the signal section and the associated zero line, and to normalize this area value to the peak-to-peak value of the third type and thus create a normalized area value,
and further comprising
a morphology discrimination unit (92), which is connected to the morphology detection unit (90) and designed to compare a respective normalized area value to at least one stored reference area value and to categorize the respective normalized area value as supraventricular tachycardia or ventricular tachycardia, depending on the magnitude of the difference between the respective normalized area value and the stored reference area value.

**6.** The signal processing device according to claim 5, **characterized in that** the morphology discrimination unit (92) is designed to determine the difference between the respective normalized area value and the stored reference area value based on an absolute difference between the current area value and the reference area value, divided by the reference area value.

**7.** A signal processing device according to claim 1 and claim 5, **characterized in that** the signal processing device is designed to average a morphological distance from the distance between the respective morphology vector and the reference vector, and the difference between the respective normalized area value and the stored reference area value, and to compare this morphological distance to a decider threshold.

**8.** The signal processing device according to claim 7, **characterized in that** the morphology discrimination unit (92) is designed to categorize a signal section as ventricular tachycardia when the morphological distance from the reference vector associated with a sinus rhythm and from the reference area is greater than the respective value of the decider threshold.

**9.** The signal processing device according to claim 7 or 8, **characterized in that** the signal processing device is designed to automatically update the decider threshold.

**10.** The signal processing device according to claim 1 or 5, **characterized in that** the signal processing device is designed to evaluate not only a signal morphology, but also a respective characteristic value identifying a particular heart rhythm.

**11.** A cardioverter/defibrillator, comprising a signal processing device according to any one of claims 1 to 10.

**12.** The cardioverter/defibrillator according to claim 11, **characterized in that** the cardioverter/defibrillator is a single-chamber cardioverter/defibrillator.

**Revendications**

1. Dispositif de traitement de signal pour classer des signaux intracardiaques, comprenant une entrée de signal pour un signal physiologique, en particulier un électrocardiogramme, et une unité de détection de morphologie (90), qui est au moins indirectement connectée à l'entrée de signal et conçue

pour détecter des maxima de signal et des minima de signal dans une section de signal et pour déterminer une valeur de crête qui est proportionnelle à l'amplitude de l'amplitude de crête pour chacun d'un nombre prédéterminé de maxima de signal et de minima de signal; et

pour déterminer une valeur crête-à-crête d'un premier type à partir de deux valeurs de crête adjacentes ayant des polarités opposées en tant que somme des valeurs absolues des valeurs de crête individuelles; et

pour déterminer, dans la même section de signal, des valeurs additionnelles crête-à-crête du premier type en tant que somme des valeurs absolues de valeurs de crête adjacentes ayant des polarités opposées; et

pour normaliser les valeurs crête-à-crête à la plus grande des valeurs crête-à-crête qui ont été déterminées et créer ainsi un nombre prédéterminé de valeurs crête-à-crête normalisées, qui ensemble conduisent à un vecteur de morphologie, lequel est caractéristique de la morphologie d'une section de signal particulière, et comprenant en outre une unité de discrimination de morphologie (92), qui est connectée à l'unité de détection de morphologie (90) et est conçue pour comparer un vecteur de morphologie respectif à au moins un vecteur de référence stocké par le fait que l'unité de discrimination de morphologie (92) identifie une distance entre le vecteur de morphologie respectif (92) et le vecteur de référence, et pour catégoriser un vecteur de morphologie respectif en tant que tachycardie supraventriculaire ou tachycardie ventriculaire, en fonction de la distance à partir du vecteur de référence.

2. Dispositif de traitement de signal selon la revendication 1, **caractérisé par le fait que** l'unité de détection de morphologie (90) est conçue

pour déterminer des valeurs crête-à-crête additionnelles d'un second type en tant que différence entre les valeurs absolues de valeurs de crête adjacentes ayant la même polarité.

3. Dispositif de traitement de signal selon l'une des revendications 1 ou 2, **caractérisé par le fait que** le dispositif de traitement de signal est conçu pour stocker automatiquement un vecteur de morphologie, selon les besoins, en tant que vecteur de référence lorsque le dispositif de traitement de signal a détecté que la section de signal associée au vecteur de morphologie respectif est un segment d'un électrocardiogramme lorsqu'un rythme sinusal normal est présent.

4. Dispositif de traitement de signal selon l'une des revendications 1 ou 2, **caractérisé par le fait que** l'unité de discrimination de morphologie (92) est conçue pour déterminer la distance entre le vecteur de morphologie respectif et le vecteur de référence en tant que distance euclidienne.

5. Dispositif de traitement de signal pour classer des signaux intracardiaques, comprenant une entrée de signal pour un signal physiologique, en particulier un électrocardiogramme, et une unité de détection de morphologie (90), qui est au moins indirectement connectée à l'entrée de signal et conçue

pour détecter les amplitudes du maximum de signal absolu et du minimum du signal absolu à l'intérieur d'une section de signal, et pour déterminer une valeur crête-à-crête d'un troisième type à partir de la différence de celles-ci; et

pour déterminer une valeur de surface qui est proportionnelle à une dimension de surface absolue enfermée par la section de signal et la ligne zéro associée, et pour normaliser cette valeur de surface à la valeur de crête-à-crête du troisième type et créer ainsi une valeur de surface normalisée,

et comprenant en outre

une unité de discrimination de morphologie (92), qui est connectée à l'unité de détection de morphologie (90) et conçue pour comparer une valeur de surface normalisée respective à au moins une valeur de surface de référence stockée et pour catégoriser la valeur de surface normalisée respective en tant que tachycardie supra-ventriculaire ou tachycardie ventriculaire, en fonction de l'amplitude de la différence entre la valeur de surface normalisée respective et la valeur de surface de référence stockée.

6. Dispositif de traitement de signal selon la revendication 5, **caractérisé par le fait que** l'unité de discrimination de morphologie (92) est conçue pour déterminer la différence entre la valeur de surface normalisée respective et la valeur de la surface de référence stockée sur la base d'une différence absolue entre la valeur de surface courante et la valeur de surface de référence, divisée par la valeur de surface de référence.

7. Dispositif de traitement de signal selon la revendication 1 et la revendication 5, **caractérisé par le fait que** le dispositif de traitement de signal est conçu pour prendre la moyenne d'une distance morphologique à partir de la distance entre le vecteur de morphologie respectif et le vecteur de référence, et de la différence entre la valeur de surface normalisée respective et la valeur de surface de référence stockée, et pour comparer cette distance morphologique à un seuil de décideur.

8. Dispositif de traitement de signal selon la revendication 7, **caractérisé par le fait que** l'unité de discrimination de morphologie (92) est conçue pour catégoriser une section de signal en tant que tachycardie ventriculaire lorsque la distance morphologique à partir du vecteur de référence associé à un rythme sinusal et à partir de la surface de référence est supérieure à la valeur respective du seuil de décideur.

9. Dispositif de traitement de signal selon l'une des revendications 7 ou 8, **caractérisé par le fait que** le dispositif de traitement de signal est conçu pour mettre à jour automatiquement le seuil de décideur.

10. Dispositif de traitement de signal selon l'une des revendications 1 ou 5, **caractérisé par le fait que** le dispositif de traitement de signal est conçu pour évaluer non seulement une morphologie de signal, mais encore une valeur caractéristique respective identifiant un rythme cardiaque particulier.

11. Défibrillateur à synchronisation automatique/défibrillateur, comprenant un dispositif de traitement de signal tel que défini à l'une quelconque des revendications 1 à 10.

12. Défibrillateur à synchronisation automatique/défibrillateur selon la revendication 11, **caractérisé par le fait que** le défibrillateur à synchronisation automatique/défibrillateur est un défibrillateur à synchronisation automatique/défibrillateur à chambre unique.

FIG. 1

**FIG. 2**

FIG. 3

FIG. 4

**FIG. 5**

FIG. 6

$$x = \text{Area / peak to peak}$$

**FIG. 7**

Fig. 8

**Fig. 9**

Fig. 10

Fig. 11

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0848965 A